# EUROPEAN PATENT APPLICATION

(11) **EP 2 433 606 A1**
(43) Date of publication of application: **28.03.2012**
(21) Application number: 11182031.2
(22) Date of filing: 20.09.2011
(51) Int. Cl.: A61H 33/00, A61H 33/06

(54) **A movable and openable panel, to be pivoted on a side thereof, for steam saunas and showers, with an optimized hydraulic distribution**

(30) Priority: 27.09.2010 IT MI20101754
(71) Applicant: Megius S.P.A., 35035 Mestrino, Padova (IT)
(72) Inventor: Mascheroni, Luigi, I-35035 MESTRINO (PD) (IT)
(74) Representative: Cicogna, Franco

(57) **Abstract**

A movable panel for steam saunas and showers, with an optimized hydraulic distribution, characterized in that said movable panel comprises a counter-frame arranged at a recess formed in a shower box or a like construction, said recess including a hydraulic device controlling a blowing device arranged above said panel or offset to a center thereof, but on the construction ceiling, said panel, which is vertically or horizontally pivoted to said counter-frame to be opened as a door, comprising all the functional operating controls of the construction.

## Description

### BACKGROUND OF THE INVENTION

The present invention relates to a movable panel for steam saunas and showers, with an optimized hydraulic distribution.

As it is known, conventional shower boxes usually comprise a hydraulic delivery assembly arranged between a side of the box and the bath room wall.

With such an arrangement, as the hydraulic delivery assembly must be subjected to repairing or servicing operations, it would be necessary to disassemble the shower box with a great time consume and disadvantages since the bath room could not be used in such an event.

The above problem has been already satisfactorily solved by the same Applicant, by a novel shower box comprising a movable inner panel defining a space for housing therein the hydraulic delivery assembly, said panel being adapted to be opened for accessing the hydraulic delivery assembly.

The above construction has made possible to access all the hydraulic delivery assemblies without the need of disassembling the shower box.

Moreover, said prior construction allowed to conceal from the view all the hydraulic delivery assembly components.

### SUMMARY OF THE INVENTION

The aim of the present invention is to provide a movable and openable panel, adapted to be pivoted or hinged on a side thereof, and which is further improved with respect to like prior movable panels.

Within the scope of the above mentioned aim, an object of the invention is to provide such a movable panel which may be easily opened owing to its pivoted connection on a single side thereof, and including both vertical and horizontal pivot pins and adapted to be opened as a door, thereby providing a multiple function construction, adapted to operate both as a steam sauna and a shower, as well as to provide yet other functions and which, moreover, comprises an improved hydraulic delivery or distributing system.

Another object of the present invention is to provide such a door-like opened panel system allowing to access the hydraulic assembly in an easier manner than prior movable panels.

Another object of the present invention is to provide such a panel including a plurality of control or drive functions.

Yet another object of the present invention is to provide an operatively flexible panel which may be fitted to different contingent operating requirements.

Another object of the present invention is to provide such a movable which, owing to its specifically designed constructional features, is very reliable and safe in operation.

According to one aspect of the present invention, the above mentioned aim and objects, as well as yet other objects, which will become more apparent hereinafter, are achieved by a movable panel for steam saunas and showers, with an optimized hydraulic distribution, characterized in that said movable panel comprises a counter-frame arranged at a recess formed in a shower box or a like construction, said recess including a hydraulic device controlling a blowing device arranged above said panel or offset to a center thereof, but on the construction ceiling, said panel, which is vertically or horizontally pivoted to said counter-frame to be opened as a door, comprising all the functional operating controls of the construction.

### BRIEF DESCRIPTION OF THE DRAWINGS

Further characteristics and advantages of the present invention will become more apparent hereinafter from the following disclosure of a preferred, though not exclusive, embodiment of the invention, which is illustrated, by way of an indicative, but not limitative, example in the accompanying drawings, where:

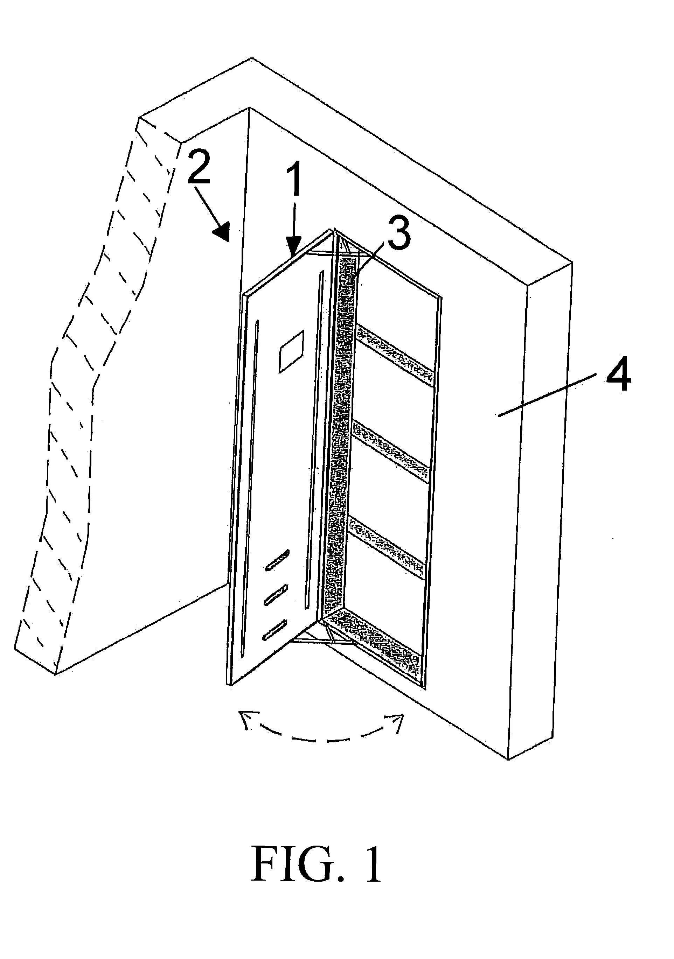

Figure 1 is a schematic perspective view of the inventive panel assembled in a shower and being shown in an open position thereof;
Figure 2 is a further perspective view similar to figure 1 but showing the panel in a closed operating position;
Figure 3 is yet another perspective view showing a further embodiment of the inventive panel, including different operating functions;
Figure 4 is yet another perspective view, similar to figure 3, but showing a further possible modified embodiment of the panel;
   and
Figure 5 is yet another perspective view showing the panel assembled on a counter-frame thereby projecting from a coating therefor.

### DESCRIPTION OF THE PREFERRED EMBODIMENTS

With reference to the number references of the above mentioned figures, the movable panel according to the present invention, generally indicated by the reference number 1, is mounted by a counter-frame 3.

The panel 1 may be easily opened, such as a door, since it is pivoted to said counter-frame.

The hydraulic device, not shown in the figures, operatively drives the blower assembly 5, which may be arranged above the panel 1 or offset to the center, but on a roof of the construction to allow a user to shower himself/herself.

According to the present invention, the panel 1 comprises all the required operating functions.

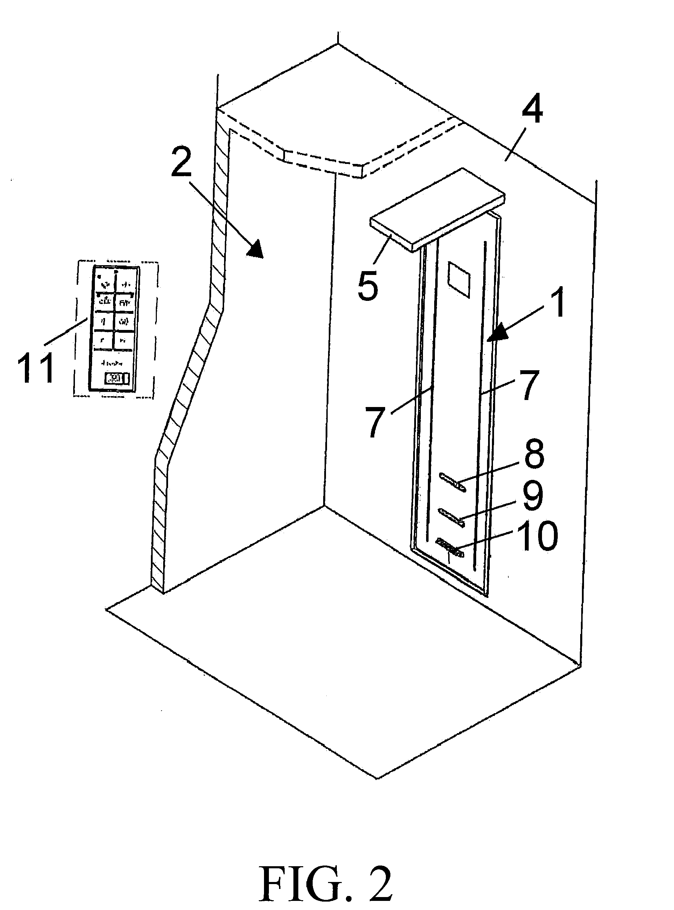

In figure 2 a panel 1 is shown comprising a multifunctional blowing device 5, of a rain, cascade or atomizing blowing type.

Said panel 1 comprises moreover chromotherapy lamps 7 preferably of a LED type, and arranged in two parallel vertical rows for performing a RGB chromotherapy method, an air suction mouth 8, a hot air diffuser 9 and a steam diffuser 10.

The panel 1 shown in figure 2 comprises moreover a water temperature (hot-cold) electronic control and a water outlet flow rate electronic control.

Said movable panel 1 comprises moreover all the function of a Turkish bath, a ventilating heating device and a radio or other sound reproducing system with a related system control arrangement.

The panel 1 of figure 2 may also comprise an outer keyboard 11 for controlling chromotherapy, Turkish bath switching on and radio program functions.

The LED's applied to said panel are designed to be switched on thereby providing constant or time variable colors.

The outer keyboard comprises moreover a USB socket.

The functions of the panels are managed by a LCD display of a "touchless" technology type.

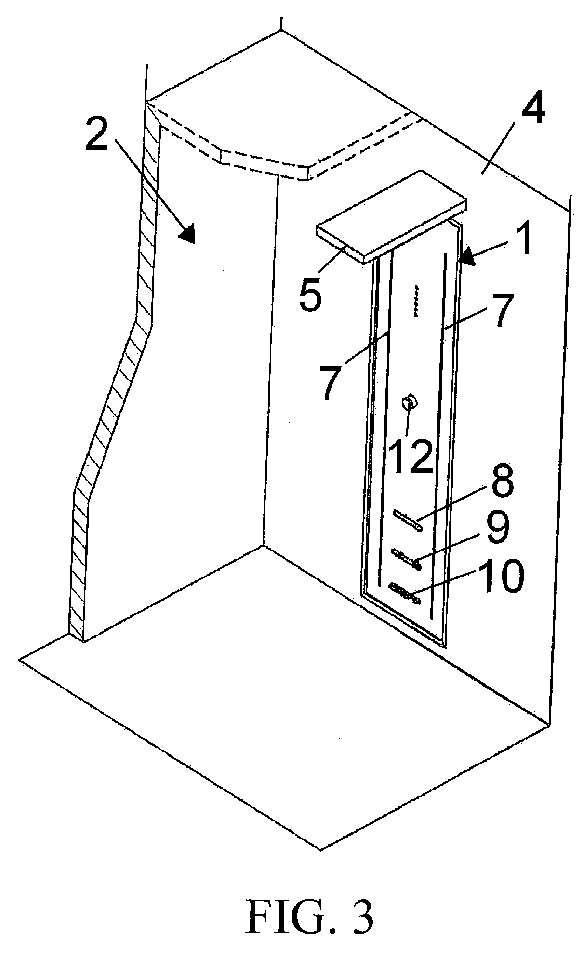

Figure 3 shows a panel 1 comprising a multifunction blowing device 5, a rain, rainfall and atomizing shower assembly.

Said panel comprises moreover a plurality of lamps 7, preferably of a LED type, arranged in two vertical parallel rows for performing a RGB chromotherapy method, an air suction mouth 8 and a hot air diffuser 9 as well as a steam diffuser 10.

The panel 1 shown in figure 3 comprises a thermostatic mixing device 12 and the Turkish bath and ventilated heating function controls.

The functions on the panel are managed or controlled through a keyboard, of a capacitive type and a "touchless" technology.

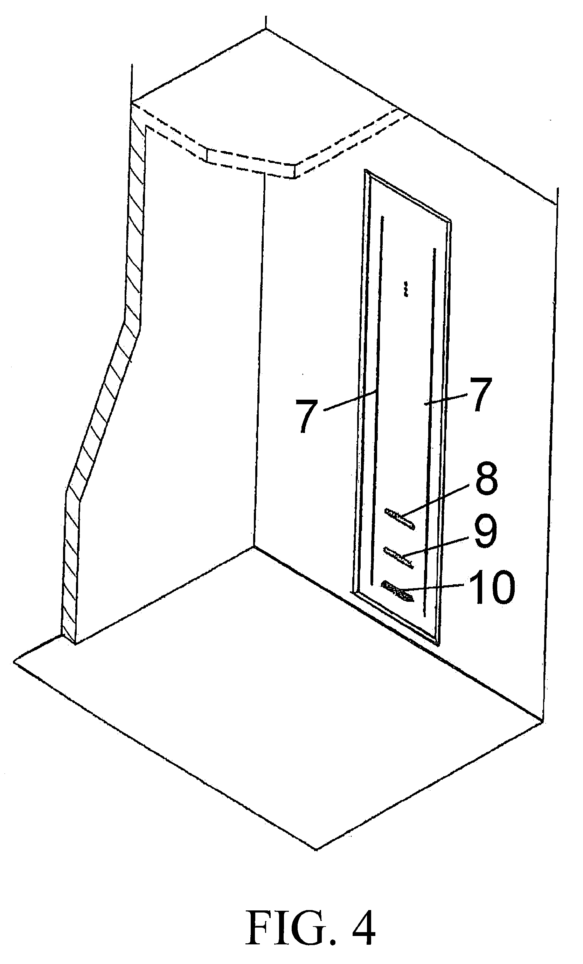

Figure 4 shows a panel 1 comprising a plurality of lamps 7, preferably of a LED type, arranged in two parallel vertical rows for performing a RGB chromotherapy method, a air suction mouth 8, a hot air diffuser 9 and a steam diffuser 10.

The panel 1 shown in figure 3 comprises a thermostatic mixing device 12 and the Turkish bath and ventilated heating operating function controls.

The panel functions are managed or controlled through a capacitive keyboard, of a "touchless" technology type.

In figures 2-4 the panel 1 is mounted flush with the wall coating 4.

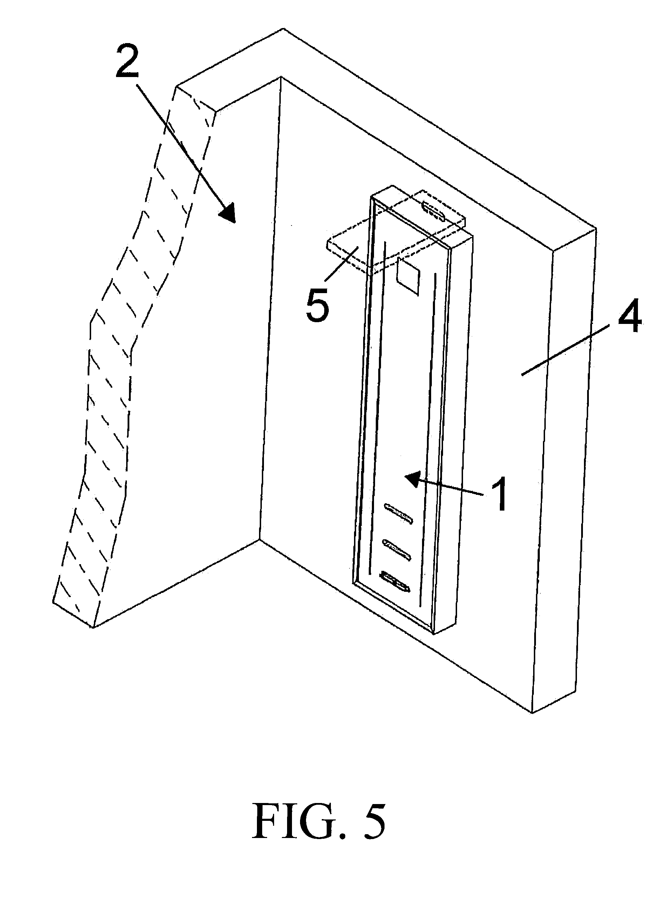

As shown in figure 5, the panel 1 is so assembled as to be pivoted to a counter-frame, on horizontal and vertical pivot pins.

It has been found that the invention fully achieves the intended aim and objects.

In fact, the invention has provided a movable multi-function panel to be applied to a steam sauna and shower construction, with an optimized hydraulic distribution.

Advantageously, the movable panel according to the present invention is connected to a counter-frame which is mounted in the space and on a wall the hydraulic device is assembled in.

This allows the panel, holding therein all the construction functional control elements to be quickly and easily assembled.

Another advantage of the present invention is that said panel may be made according to several modified embodiments thereby providing an operatively flexible product adapted to meet a lot of different operating requirements.

In practicing the invention, the used materials, as well as the contingent size and shapes, can be any, depending on requirements.

## Claims

1. A movable panel for steam saunas and showers, with an optimized hydraulic distribution, **characterized in that** said movable panel comprises a counter-frame arranged at a recess formed in a shower box or a like construction, said recess including a hydraulic device controlling a blowing device arranged above said panel or offset to a center thereof, but on the construction ceiling, said panel, which is vertically or horizontally pivoted to said counter-frame to be opened as a door, comprising all the functional operating controls of the construction.

2. A movable panel, according to claim 1, **characterized in that** said movable panel comprises a multifunction blowing device, and a rain, fall and nebulizing system.

3. A movable panel, according to claim 1, **characterized in that** said movable panel comprises chromotherapy lamps.

4. A movable panel, according to claim 3, **characterized in that** said chromotherapy lamps are of a LED type and are arranged in two parallel vertical rows for performing a RGB chromotherapy method, said LED's being adapted to be lighted so as to show constant or time variable colors.

5. A movable panel, according to one or more of the preceding claims, **characterized in that** said movable panel comprises an air suction mouth, a hot air diffuser and a steam diffuser.

6. A movable panel, according to one or more of the preceding claims, **characterized in that** said movable panel comprises a water temperature electronic control and a water outlet flow rate electronic control.

7. A movable panel, according to one or more of the preceding claims, **characterized in that** said movable panel comprises all the functions of a Turkish bath and a ventilating heating device.

8. A movable panel, according to one or more of the preceding claims, **characterized in that** said movable panel comprises a radio or other sound reproducing system with a related system control arrangement.

9. A movable panel, according to one or more of the preceding claims, **characterized in that** said movable panel comprises an outer keyboard for controlling chromotherapy, Turkish bath switching on and radio program functions, said outer keyboard comprising moreover a USB socket.

10. A movable panel, according to one or more of the preceding claims, **characterized in that** the functions on said panel are managed by a LCD display or a capacitive keyboard of a "touchless" technology type.
